# EUROPEAN PATENT APPLICATION

(11) **EP 0 628 638 A2**
(43) Date of publication of application: **14.12.1994**
(21) Application number: 94303654.1
(22) Date of filing: 23.05.1994
(51) Int. Cl.: C12P 13/04, C12N 15/54

(54) **Process for preparing 2-amino-3-hydroxy acids**

(30) Priority: 25.05.1993 US 67450; 31.03.1994 US 220622
(71) Applicant: ELI LILLY AND COMPANY, Indianapolis, Indiana 46285 (US)
(72) Inventor: Dotzlaf, Joe Edward, Greenwood, Indiana 46142 (US); Gazak, Robert James, Indianapolis, Indiana 46201 (US); Kreuzman, Adam Joseph, Greenwood, Indiana 46142 (US); Kroeff, Eugene Paul, Carmel, Indiana 46033 (US); Queener, Stephen Wyatt, Indianapolis, Indiana 46208 (US); Vicenzi, Jeffrey Thomas, Indianapolis, Indiana 46220 (US); Yeh, Wu-Kuang, Greenwood, Indiana 46142 (US); Zock, Joseph Martin, Greenwood, Indiana 46143 (US)
(74) Representative: Hudson, Christopher Mark

(57) **Abstract**

The invention provides a process for preparing 2-amino-3-hydroxy acids. The process comprises mixing an aldehyde, and glycine with *Escherichia coli* serine hydroxymethyltransferase in the presence of pyridoxal 5'-phosphate to form the corresponding L-erythro-2-amino-3-hydroxy acid.

## Description

This invention relates to a process for the manufacture of 2-amino-3-hydroxy acids which have many uses, such as serving as intermediates in the preparation of antibiotics. See, for example, U.S. Patent No. 4,820,815; U.S. Patent No. 4,820,815; Mattingly et al., J. Org. Chem. 46:1557 (1981); Miller et al., J. Am. Chem. Soc. 102:7072 (1980); and Lotz, et al., 1993, J. Organic Chemistry 58:618. A number of methods for the preparation of 2-amino-3-hydroxy acids are known, however, most have one or more disadvantages. These include a lack of generality and poor stereochemical control.

Enzymatic processes have some distinct advantages over chemical processes. For example, they are carried out in aqueous systems under mild conditions and frequently are stereoselective. The enzyme employed in the process of this invention is SHMT. European Patent Publication 0460883A2, published December 11, 1991, described a process for obtaining 2-amino-3-hydroxy acids by a SHMT-catalyzed condensation of certain aldehydes with glycine. This process comprised incubating glycine and an aldehyde with SHMT in an aqueous medium at a pH of 7.0 to 8.0 and a temperature of about 30°C to 55°C.

U.S. Patent No. 5,102,792 describes a process for the production of L-erythro-serine derivatives by a SHMT-catalyzed aldol condensation of glycine and an aldehyde. The process employs an extractive process using organic solvents in conjunction with the aldol condensation reaction to provide diastereomeric specificity.

However, as described by Saeed and Young, 1992, Tetrahedron 48(12) :2507-2514, SHMT-catalyzed condensation reactions at batch preparative scale which did not employ a continuous extraction process of the product provided low stereoselectivity.

The process of the present invention allows SHMT-catalyzed aldol condensation reactions to proceed in high yield and high diastereomeric specificity directly, without the need for continuous and coupled extraction. The elimination of water immiscible solvents as extractive agents enhances the environmental safety of the process and allows a simple batch or fed-batch mode operation. The conditions of the process of the present invention are amenable to safe, practical, and economic manufacture of L-erythro-2-amino-3-hydroxy acids at large scale.

The invention provides a process for preparing a 2-amino-3-hydroxy acid of the formula 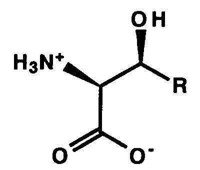
wherein R is C₃-C₆ alkenyl; C₂-C₆ aldehydo; C₃-C₆ alkynyl; C₂-C₆ alkyl substituted by esterified carboxy, C₁-C₆ alkoxy, hydroxy, halo; phenyl; or furyl, which comprises reacting at a temperature of between about 0°C and about 25°C in an aqueous solution at a pH between about 6.0 and about 8.0, an aldehyde of the formula RCHO (wherein R has the same meaning as defined above) and glycine with *Escherichia coli* serine hydroxymethyltransferase in the presence of pyridoxal 5'-phosphate.

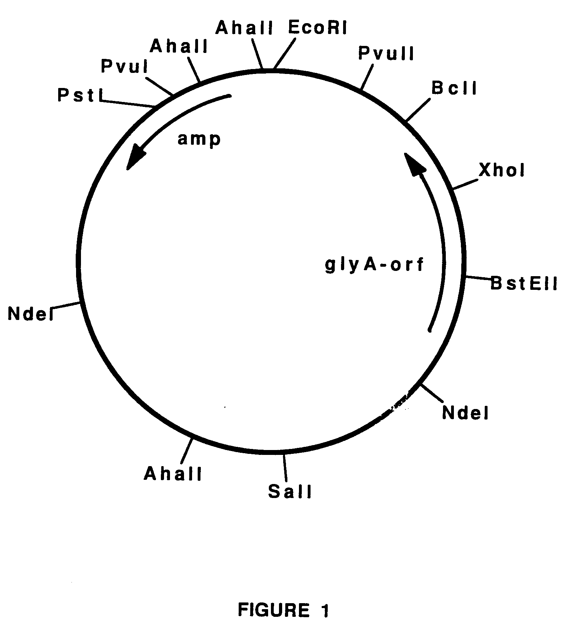

Figure 1 is a restriction enzyme site and function map of plasmid pGS29.

Figure 2 is a restriction enzyme site and function map of plasmid pHKY390.

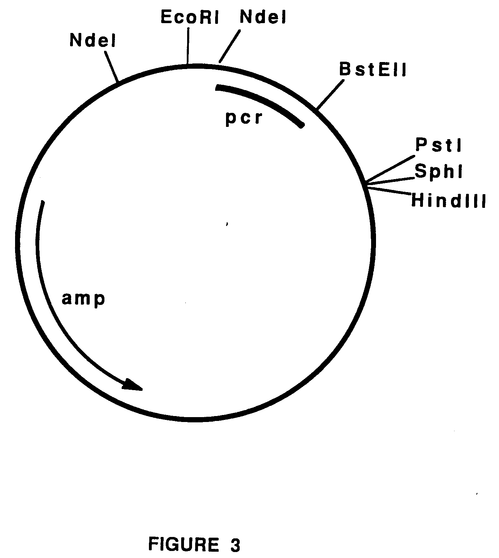

Figure 3 is a restriction enzyme site and function map of plasmid pV-pcrB.

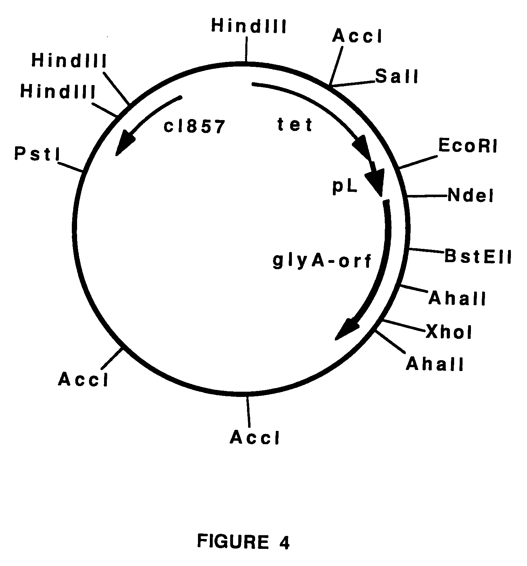

Figure 4 is a restriction enzyme site and function map of plasmid pZPl-glyA.

The restriction enzyme site and function maps presented in the drawings are approximate representations of the recombinant DNA vectors discussed herein. The restriction site information is not exhaustive, there may be more restriction enzyme sites of a given type than are actually shown on the map.

For the purposes of this invention as disclosed and claimed herein, the following items are defined.
AHHA - 2-amino-3-hydroxy-hept-6-enoic acid
amp - ampicillin resistance phenotype and gene conferring the same.
Batch mode - process wherein each reactant is added once and after a specific time the product may be isolated.
cI857 - a gene encoding a temperature sensitive repressor of the bacteriophage lambda pL promoter.
Coding sequence - the sequence of DNA in a gene that encodes the amino acid residue sequence of the protein expressed from the gene.
Fed-batch mode - process wherein at least one reactant is added multiple times in a step-wise or continuous manner and after a specific time the product may be isolated.
Gene - a segment of DNA that comprises a promoter, translational activating sequence, coding sequence, and 3' regulatory sequences, positioned to drive expression of the gene product.
kan - kanamycin resistance phenotype and gene conferring the same.
L-erythro-AHHA - 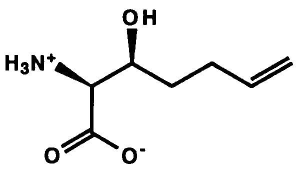
pL - leftward promoter from bacteriophage lambda.
PLP - pyridoxal 5'-phosphate.
Promoter - a DNA sequence that directs or initiates the transcription of DNA.
SHMT - *Escherichia coli* serine hydroxymethyltransferase.
tet - tetracycline resistance phenotype and gene conferring the same.

The process of this invention provides for the preparation of 2-amino-3-hydroxy acids which comprises mixing an appropriate aldehyde with a buffered solution of glycine, pyridoxal 5'-phosphate, and SHMT under conditions that allow aldol condensation of glycine with the aldehyde to form the corresponding L-erythro-2-amino-3-hydroxy acid in high yield and with high diastereomeric specificity. The ability to form high concentrations of L-erythro-2-amino-3-hydroxy acids in simple batch or fed-batch mode with high diastereomeric specificity without need for extraction with organic solvents in a SHMT-catalyzed aldol condensation makes the present invention especially useful for large scale operations. Under fed-batch conditions, the process of the present invention provides greater than 75% conversion of the aldehyde to the corresponding 2-amino-3-hydroxy acid of which greater than 90% is the L-erythro diastereomer form.

The pH of the process of this invention is maintained between about pH 6.0 and pH 8.0. More preferably, the pH of the reaction is maintained between pH 6.5 and 7.5. Most preferably, the reaction is maintained at pH 7.0. Phosphate buffers or continual pH adjustment with acid and/or base are suitable for providing the desired pH range.

The relative proportions of glycine and the aldehyde used in the enzymatically catalyzed aldol condensation may vary. However, higher yields of the L-erythro diastereomer product from the aldehyde are obtained when a higher ratio of glycine to aldehyde is employed. Preferably, the glycine concentration is in excess while the aldehyde concentration is kept to a minimum by slow, continuous addition such that the final ratio of glycine to aldehyde is from about 10:1 to about 2:1. Preferably, the final ratio of glycine to aldehyde is about 4:1. Preferred glycine concentrations are from about 0.5 M to about 3 M. For example, 1 M glycine and 250 mM aldehyde can be utilized in the reaction. This ratio is preferably obtained by the fed-batch mode with the continuous or step-wise addition of the aldehyde to the reaction mixture with stirring sufficient to rapidly disperse the aldehyde to a homogeneous solution in the reaction mixture. The concentration of the aldehyde is minimized because high concentrations of aldehyde in the reaction mixture accelerate inactivation of the SHMT enzyme.

The duration of the reaction is the minimal time required to convert greater than about 75% of the total aldehyde added to the reaction to the corresponding 2-amino-3-hydroxy acid. Attempts to exceed these conversion percentages by extending the reaction time may result in lower yields and increased levels of undersired threo-2-amino-3-hydroxy acid product. The duration of the reaction may be varied inversely proportional to the amount of SHMT added and temperature of incubation. For example, with 100 µM SHMT at 15°C, 1 M glycine, and 250 mM aldehyde, the duration of the reaction will be about 4 hours, as compared to a reaction time of 3 hours when 100 µM of SHMT and a temperature of 20°C.

As noted above, the process of this invention can be carried out in fed-batch mode or batch-mode. Using the fed-batch mode, which is preferred, the aldehyde may be added at a predetermined rate over the duration of the reaction so that its concentration in the reaction is minimized. The rate of addition of the aldehyde can be constant or decreasing over time.

The aldehyde may also be added in batch mode. However, the higher concentration of aldehyde present early in the reaction will lessen the number of times the SHMT enzyme can be recycled for reuse in batch mode because of an undesirable side-reaction of aldehyde with SHMT that accelerates with increasing concentrations of aldehyde. Using batch mode conditions, the yield of glycine and aldehyde to 2-amino-3-hydroxy acid is lower than the fed-batch mode. For example, when 250 mM 4-pentenal is reacted with 1 M glycine and 100 µM SHMT at 15°C under batch mode conditions, the conversion to 2-amino-3-hydroxy-hept-6-enoic acid was about 50%.

The amount of SHMT used in the reaction may vary. However, the SHMT concentration is preferably between 10 µM and 1 mM. More preferably, the SHMT concentration is about 50-150 µM. Most preferably, the SHMT concentration is about 100 µM. As the concentration of SHMT is lowered, an undesired non-enzymatic chemical reaction between the aldehyde and glycine lowers yield. References to SHMT concentration herein refers to active SHMT as determined by the enzyme assay of Example 5.

As with certain other enzymatic reactions, cofactors influence the substrate specificity, stability, and catalytic efficiency of SHMT. An essential cofactor in the process of this invention is pyridoxal 5'-phosphate (PLP). In the process of the present invention, PLP is provided in the reaction in about 2-15 fold molar excess relative to SHMT. Preferably PLP is provided in about 10-fold molar excess of SHMT. The enzyme is more stable and can be reused more times with PLP in excess.

The process of this invention is carried out at a temperature between about 0°C and 25°C. SHMT is more stereoselective at such lower temperatures. Thus, lower temperature provides for greater production of the L-erythro-2-amino-3-hydroxy acid product. Below 10°C, higher amounts of SHMT, up to 1 mM at 0°C, are required to maintain an acceptable rate of aldol condensation between glycine and aldehyde. Above about 20°C, a loss in yield from aldehyde is observed as well as a decrease in stereoselectivity of the 2-amino-3-hydroxy acid product. Preferably, the reaction is carried out at about 15°C.

As noted above, a wide variety of aldehydes may be used in the process of this invention. These aldehydes, of the formula RCHO, include those compounds wherein R is C₃-C₆ alkenyl; C₂-C₆ aldehydo; C₃-C₆ alkynyl; C₂-C₆ alkyl substituted by esterified carboxy, C₁-C₆ alkoxy, hydroxy, halo, or cyano; phenyl; or furyl.

As used herein the term C₃-C₆ alkenyl refers to straight and branched unsaturated hydrocarbon chains where the carbon to carbon double bond is not conjugated to the aldehyde moiety such as propenyl, butenyl, pentenyl and hexenyl; C₃-C₆ alkynyl refers to straight and branched unsaturated hydrocarbon chains where the carbon to carbon triple bond is not conjugated to the aldehyde moiety such as propynyl, butynyl, pentynyl and hexynyl groups which may be branched; C₁-C₆ alkyl substituted by esterified carboxy refers to a straight and branched chain alkyl groups substituted by an esterified carboxy group wherein the ester group is C₁-C₄ alkyl, phenyl, benzyl, substituted benzyl such as p-methoxybenzyl, methylbenzyl, p-nitrobenzyl, diphenylmethyl, or other conventional carboxy protecting group. Examples of such groups are ethoxycarbonylmethyl, 2(methoxycarbonyl)-ethyl, 3-(t-butyloxycarbonyl)propyl, 3-(benzyloxycarbonyl)butyl, 5-(4-methoxybenzyloxycarbonyl) hexyl, and like alkyl groups substituted by esterified carboxy groups. C₂-C₆ alkyl substituted by C₁-C₆ alkoxy refers to, e.g., 2-methoxyethyl, 3-methoxypropyl, 4-ethoxybutyl, 5-pentoxyhexyl, 6-propoxyhexyl, and the like. C₂-C₆ alkyl substituted by hydroxy refers to, e.g., 2-hydroxyethyl, 4-hydroxybutyl, 3-hydroxyhexyl, and the like. C₂-C₆ alkyl substituted by halo refers to, e.g., 2-chloroethyl, 3-fluoropropyl, 3-iodobutyl, 5-bromohexyl, and the like. C₂-C₆ alkyl substituted by cyano refers to, e.g., 2-cyanoethyl, 3-cyanopropyl, 3-cyanobutyl, 5-cyanohexyl, and the like. C₂-C₆ aldehydo refers to groups such as 3-formyl propyl.

An especially preferred aldehyde for use in the invention as described herein is 4-pentenal. The product of the aldol condensation of glycine and 4-pentenal is L-erythro-2-amino-3-hydroxy-hept-6-enoic acid.

Another preferred aldehyde for use in the invention is 3-cyanopropanal. The product of the aldol condensation of glycine and 3-cyanopropanal is 2-amino-3-hydroxy-5-cyano-pentanoic acid (AHCPA).

In another preferred embodiment of this invention an ester of succinic semialdehyde such as C₁-C₄ alkyl ester, for example, the methyl ester, ethyl ester or butyl ester, is fed to and incubated with a reaction mixture substantially as described above to yield the corresponding L-erythro-2-amino-3-hydroxy adipic acid. The monoester product of the enzymatic reaction spontaneously converts to a lactone, which in the presence of base, or higher temperatures, readily converts to a dicarboxylic acid salt. Under the reaction conditions described above, the lactone form is the dominant product.

The aldehydes used in the process are all known compounds available commercially or preparable by conventional methods. It is not necessary that the aldehyde be completely in solution for its conversion to the corresponding 2-amino-3-hydroxy acid to occur. Aldehydes which are only partially soluble in the aqueous reaction medium also serve as substrates for SHMT.

The process can be monitored for production of the 2-amino-3-hydroxy acid by removing aliquots from the incubation mixture from time to time and assaying the samples by HPLC separation and detection of O-phthalaldehyde derived isoindoles of all primary amine compounds present in the mixture. The assay procedure is described by Aswad, 1984, Analytical Biochemistry 137:405. HPLC analysis of both the incubation mixture and the enzyme blank in comparison to O-phthalaldehyde derivatives of authentic product standards, allowed determination of those peaks on the chromatographs that could be assigned to enzymatic reaction products.

The SHMT enzyme source may comprise, for example, intact, whole cells containing SHMT; a clarified washed cell lysate of such cells; or partially purified or purified SHMT. Multistep techniques for recovery and purification of SHMT are well known in the art and are described, for example, by Ulevitch et al., Biochemistry 16(24) 5342-5350 (1977) and Schirch et al., J. Bacteriol. 163(1)1-7 (1985).

Thus, in one embodiment of the invention, the aldol condensation reaction is carried out in the presence of a clarified washed cell lysate of *Escherichia coli* cells transformed with a recombinant DNA expression vector which provides expression of SHMT at levels above about 40% of the total water soluble protein. A method of obtaining this level of SHMT expression is described in the accompanying examples. Direct use of the clarified washed cell lysate in the process of the present invention allows costly purification steps to be avoided.

In another embodiment of the invention, the aldol condensation reaction may be carried out in the presence of *Escherichia coli* cells transformed with a recombinant DNA expression vector which provides expression of SHMT at levels above about 40% of the total water soluble protein.

In still another embodiment of the invention, the aldol condensation may be carried out with partially purified SHMT obtained from a single anion exchange chromatography step. In this embodiment the purified SHMT is obtained from whole cells in which SHMT is obtained from *Escherichia coli* cells transformed with a recombinant DNA expression vector which provides expression of SHMT at levels above about 40% of the total water soluble protein.

Further, the SHMT enzyme or intact cell containing the SHMT enzyme may be immobilized, using a variety of supports and immobilization techniques that are well known in the art.

Another useful feature of the process of the present invention is that the SHMT enzyme can be recycled and used in subsequent reactions. When sufficient 2-amino-3-hydroxy acid product has been formed, the SHMT is recycled by forcing the reaction mixture through an ultrafilter with a cut-off of about 30,000 molecular weight. The SHMT is retained by the filter for reuse. The filtrate contains the 2-amino-3-hydroxy acid product, glycine, PLP, and unreacted aldehyde substrate. The 2-amino-3-hydroxy acid product is retained on an SP207 adsorption column (Mitsubishi Chemical Industries, Co., Ltd.) and can be selectively eluted with aqueous methanol. The SHMT retained by ultrafiltration is then recharged with glycine and PLP, the solution is adjusted to a pH of about 7.0, and aldehyde is fed to the mixture to initiate the next cycle of aldol condensation. The reaction is monitored for product until greater than about 75% of the total amount of added aldehyde is converted to the corresponding 2-amino-3-hydroxy acid, and the process is repeated.

The process of the present invention utilizes high concentrations of SHMT. Stauffer et al., 1981, Gene 14:63-72, describe the cloning of the *gly*A gene which encodes SHMT in *E. coli.* The nucleotide sequence for the *gly*A gene has been determined and the amino acid sequence for the SHMT enzyme has been proposed by Plamann et al., 1983, Nucleic Acid Research 11:2065-2075. The *gly*A gene has been overexpressed in *E. coli* host cells at a level 17-26 fold over the wild type and the overproduced enzyme has been isolated and purified (Schirch et al., 1985, J. Bacteriol. 163 (1) :1-7) . However, this level of expression is not practical for large scale production of 2-amino-3-hydroxy amino acids. To provide a commercially feasible method of obtaining the large amount of SHMT needed to run the process of the present invention at large scale, a system for the heterologous production of SHMT by recombinant DNA technology is also provided by the examples below. The process of producing SHMT described herein is identical to that which is disclosed in Attorney Docket X-9260 entitled "Process for Preparing Serine Hydroxymethyltransferase", which was filed on the same day as the present application. The entire contents of Attorney Docket X-9260 is incorporated herein by reference.

As described below in the examples, the plasmid pGS29 was the source of the DNA encoding the *Escherichia coli gly*A gene in the construction of the vectors of the present invention. As an alternative to the cloning scheme presented below, the DNA sequence encoding *E. coli gly*A may be prepared for use in the vectors of the present invention by a variety of other methods, including DNA synthesis, cDNA cloning, genomic cloning, polymerase chain reaction (PCR) technology, or a combination of these approaches. These and other techniques are described by Maniatis, et al. Molecular Cloning: A Laboratory Manual, Cold Spring Harbor Press, Cold Spring Harbor Laboratory, Cold Spring Harbor, New York (1989), or Current Protocols in Molecular Biology (F. M. Ausbel et al., 1989). The contents of both of these references are incorporated herein by reference.

The DNA sequences of *Escherichia coli gly*A open reading frame can be synthesized using commercially available methods and equipment. For example, the solid phase phosphotriester method can be used to produce the DNA sequences of this invention. The DNA sequences can be synthesized by the modified phosphotriester method using fully protected DNA building blocks. Such synthetic methods can be carried out in substantial accordance with the procedure of Itakura, et al., 1977, Science 198:1056 and Crea, et al., Proc. Natl. Acad. Sci. U.S.A. 75:575, and Narang, et al., 1980, Methods in Enzymology 68:90. In addition to manual procedures, the DNA sequences can be synthesized using automated synthesizers such as the ABS 380A DNA Synthesizer (Applied Biosystems, 850 Lincoln Centre Drive, Foster City, CA 94404). The DNA sequence can also be generated by the polymerase chain reaction. See, for example, United States Patent Nos. 4,800,159 and 4,683,202, and European Patent Publication No. 0258017, published March 2, 1987.

For example, the following DNA sequence which encodes the *Escherichia coli gly*A open reading frame and has NdeI and BclI compatible ends can be synthesized and cloned into the large NdeI-BamHI DNA fragment of plasmid pHKY390 to create plasmid pZPl-glyA. The 5' to 3' strand of the following double stranded DNA fragment is designated SEQ. ID. NO 1. The 3' to 5' strand of the following double stranded DNA fragment is designated SEQ. ID. NO 2.

5'-T ATG TTA AAG CGT GAA ATG AAC ATT GCC GAT TAT GAT
3'-   AC AAT TTC GCA CTT TAC TTG TAA CGG CTA ATA CTA

GCC GAA GTC TGG CAG GCT ATG GAG CAG GAA AAA GTA CGT CAG
CGG CTT CAG ACC GTC CGA TAC CTC GTC CTT TTT CAT GCA GTC

GAA GAG CAC ATC GAA CTG ATC GCC TCC GAA AAC TAC ACC AGC
CTT CTC GTG TAG CTT GAC TAG CGG AGG CTT TTG ATG TGG TCG

CCC CGC GTA ATG CAG GCG CAG GGT TCT CAG CTG ACC AAC AAA
GGG GCG CAT TAC GTC CGC GTC CCA AGA GTC GAC TGG TTG TTT

TAT GCT GAA GGT TAT CCG GGC AAA CGC TAC TAC GGC GGT TGC
ATA CGA CTT CCA ATA GGC CCG TTT GCG ATG ATG CCG CCA ACG

GAG TAT GTT GAT ATC GTT GAA CAA CTG GCG ATC GAT CGT GCG

CTC ATA CAA CTA TAG CAA CTT GTT GAC CGC TAG CTA GCA CGC

AAA GAA CTG TTC GGC GCT GAC TAC GCT AAC GTC CAG CCG CAC
TTT CTT GAC AAG CCG CGA CTG ATG CGA TTG CAG GTC GGC GTG

TCC GGC TCC CAG GCT AAC TTT GCG GTC TAC ACC GCG CTG CTG
AGG CCG AGG GTC CGA TTG AAA CGC CAG ATG TGG CGC GAC GAC

GAA CCA GGT GAT ACC GTT CTG GGT ATG AAC CTG GCG CAT GGC
CTT GGT CCA CTA TGG CAA GAC CCA TAC TTG GAC CGC GTA CCG

GGT CAC CTG ACT CAC GGT TCT CCG GTT AAC TTC TCC GGT AAA
CCT GTG GAC TGA GTG CCA AGA GGC CAA TTG AAG AGG CCA TTT

CTG TAC AAC ATC GTT CCT TAC GGT ATC GAT GCT ACC GGT CAT
GAC ATG TTG TAG CAA GGA ATG CCA TAG CTA CGA TGG CCA GTA

ATC GAC TAC GCC GAT CTG GAA AAA CAA GCC AAA GAA CAC AAG
TAG CTG ATG CGG CTA GAC CTT TTT GTT CGG TTT CTT GTG TTC

CCG AAA ATG ATT ATC GGT GGT TTC TCT GCA TAT TCC GGC GTG
GGC TTT TAC TAA TAG CCA CCA AAG AGA CGT ATA AGG CCG CAC

GTG GAC TGG GCG AAA ATG CGT GAA ATC GCT GAC AGC ATC GGT
CAC CTG ACC CGC TTT TAC GCA CTT TAG CGA CTG TCG TAG CCA

GCT TAC CTG TTC GTT GAT ATG GCG CAC GTT GCG GGC CTG GTT
CGA ATG GAC AAG CAA CTA TAC CGC GTG CAA CGC CCG GAC CAA

GCT GCT GGC GTC TAC CCG AAC CCG GTT CCT CAT GCT CAC GTT
CGA CGA CCG CAG ATG GGC TTG GGC CAA GGA GTA CGA GTG CAA

GTT ACT ACC ACC ACT CAC AAA ACC CTG GCG GGT CCG CGC GGC
CAA TGA TGG TGG TGA GTG TTT TGG GAC CGC CCA GGC GCG CCG

GGC CTG ATC CTG GCG AAA GGT GGT AGC GAA GAG CTG TAC AAA
CCG GAC TAG GAC CGC TTT CCA CCA TCG CTT CTC GAC ATG TTT

AAA CTG AAC TCT GCC GTT TTC CCT GGT GGT CAG GGC GGT CCG
TTT GAC TTG AGA CGG CAA AAG GGA CCA CCA GTC CCG CCA GGC

TTG ATG CAC GTA ATC GCC GGT AAA GCG GTT GCT CTG AAA GAA
AAC TAC GTG CAT TAG CGG CCA TTT CGC CAA CGA GAC TTT CTT

GCG ATG GAG CCT GAG TTC AAA ACT TAC CAG CAG CAG GTC GCT
CGC TAC CTC GGA CTC AAG TTT TGA ATG GTC GTC GTC CAG CGA

AAA AAC GCT AAA GCG ATG GTA GAA GTG TTC CTC GAG CGC GGC
TTT TTG CGA TTT CGC TAC CAT CTT CAC AAG GAG CTC GCG CCG

TAC AAA GTG GTT TCC GGC GGC ACT GAT AAC CAC CTG TTC CTG
ATG TTT CAC CAA AGG CCG CCG TGA CTA TTG GTG GAC AAG GAC

GTT GAT CTG GTT GAT AAA AAC CTG ACC GTT AAA GAA GCA GAC
CAA CTA GAC CAA CTA TTT TTG GAC TAA CAA TTT CTT CGT CTG

GCC GCT CTG GGC CGT GCT AAC ATC ACC GTC AAC AAA AAC AGC
CGG CGA GAC CCG GCA CGA TTG TAG TGG CAG TTG TTT TTG TCG

GTA CCG AAC GAT CCG AAG AGC CCG TTT GTG ACC TCC GGT ATT
CGT GGC TTG CTA GGC TTC TCG GGC AAA CAC TGG AGG CCA TAA

CGT GTA GGT ACT CCG GCG ATT ACC CGT CGC GGC TTT AAA GAA
GCA CAT CCA TGA GGC CGC TAA TGG GCA GCG CCG AAA TTT CTT

GCC GAA GCG AAA GAA CTG GCT GGC TGG ATG TGT GAC GTG CTG
CGG CTT CGC TTT CTT GAC CGA CCG ACC TAC ACA CTG CTC GAC

GAC AGC ATC AAT GAT GAA GCC GTT ATC GAG CGC ATC AAA GGT
CTG TCG TAG TTA CTA CTT CGG CAA TAG CTC GCG TAG TTT CCA

AAA GTT CTC GAC ATC TGC GCA CGT TAC CCG GTT TAC GCA
TTT CAA GAG CTG TAG ACG CGT GCA ATG GGC CAA ATG CGT

TAAGCGAAAC GGTGATTTGC TGTCAATGTG CTCGTTGTTC ATGCCGGATG
ATTCGCTTTG CCACTAAACG ACAGTTACAC GAGCAACAAG TACGGCCTAC

CGGCGTGAAC GCCTTATCCG GCCTACAAAA CTTTGCAAAT TCAATATATT
GCCGCACTTA CGGAATAGGC CGGATGTTTT GAAACGTTTA AGTTATATAA

GCAATCTCCG TGTAGGCCTG ATAAGCGTAG CGCATCAGGC AATTTTTCGT
CGTTAGAGGC ACATCCGGAC TATTCGCATC GCGTAGTCCG TTAAAAAGCA

TTAT -3'
AATACTAG-5'

The following examples are intended to assist in the further understanding of the invention. Particular materials employed, species, and conditions are intended to be further illustrative of the invention and not limiting the reasonable scope thereof. Procedures for the manipulation and analysis of DNA were performed essentially as described by Sambrook *et al.*, 1989, *Molecular Cloning: a Laboratory Manual*, Cold Spring Harbor Laboratory Press, Cold Spring Harbor, NY. Conditions for restriction enzyme reactions were those recommended by the manufacturers (Boehringer Mannheim (BM), Indianapolis, IN; New England Biolabs (NEB), Beverly, MA; Bethesda Research Labs (BRL), Gaithersburg, MD.

### EXAMPLE 1

### Preparation of Escherichia coli Host Cells Transformed with Plasmid pGS29

The nucleotide sequence of the *Escherichia coli gly*A gene is known (Plamann et al., 1983, Nucleic Acid Research 11:2065-2075. However, as a matter of convenience, plasmid pGS29 isolated from *Escherichia coli* GS245/pGS29 was employed as a starting material for the vectors of this invention. *Escherichia coli* GS245/pGS29 was described by Plamann and Stauffer, 1983, Gene 22:9-18.

A portion of an *Escherichia coli* GS245/pGS29 colony grown on L-agar containing 50 µg/ml ampicillin was transferred to one liter of L-broth containing 50 µg/ml ampicillin and incubated in an air-shaker at 37°C for about 16 hours. Cells were harvested in 500 ml bottles in a Beckman JA-10 rotor (Beckman Insts. Inc., Fullerton, CA. 92634 (Beckman)) at 8000 rpm for 10 minutes at 4°C. The cell pellet was washed with TE-8.0 (10 mM Tris-HCl (pH 8.0) 1 mM ethylenediaminetetraacetic acid (EDTA)), collected, and resuspended in 50 mM Tris-HCl (pH 8.0) and 25% sucrose to a total volume of 10 ml. One milliliter of 20 mg/ml lysozyme (Sigma Chemical Co., St. Louis, MO 63178) in 25 mM Tris-HCl, pH 8.0, was added with stirring and the mixture was then incubated on ice for 30 minutes. Four milliliters of 200 mM EDTA was added with subsequent incubation on ice for 10 minutes followed by the addition of 15 ml of Brij/DOC lysing solution (1% Brij 58; 0.4% deoxycholate; 50 mM Tris-HCl, pH 8.0; 60 mM EDTA). Tubes were gently inverted to mix and incubated on ice for 15-30 minutes. Cell debris was removed by centrifugation for 1 hour at 18,000 rpm in a Beckman JA-20 rotor. Supernatant was decanted yielding approximately 30 ml to which was added 150 µl of 10 mg/ml RNAse A (Sigma). After a 1 hour incubation at 37°C, 150 µl of 10 mg/ml Proteinase K (Boehringer Mannheim) was added, followed by another 1 hour incubation at 37°C. DNA was precipitated by the addition of 1/10 volume of 3 M sodium acetate, pH 7.0, followed by 3X volumes of ice cold absolute ethanol. DNA was recovered by centrifugation in a Beckman JA-14 rotor at 8,000 rpm for 30 minutes. The air dried pellet was resuspended in TE-8.0 to a total volume of 9 ml, to which was added 9 g of cesium chloride (Boehringer Mannheim) and 0.5 ml of 10 mg/ml ethidium bromide. The resulting solution was loaded into two 5.1 ml Quik-seal tubes (Beckman) and centrifuged at 65,000 rpm in a Beckman VTi65.2 ultracentrifuge rotor for 6 hours. The plasmid band was visualized under ultraviolet light and was removed by syringe. The resulting DNA solution was extracted with salt-saturated isopropanol to remove the ethidium bromide and dialyzed 16 hours against 1000x volumes TE-8.0. DNA solutions were stored at -20°C until needed.

Transformation competent *Escherichia coli* DH5αcells were obtainbed from Gibco BRL (Gaithersburg, MD) and were transformed with plasmid pGS29 in accordance with the manufacturer's protocol. Transformants were selected on L-agar containing 50 µg/ml ampicillin. Transformants containing plasmid pGS29 were verified by plasmid sizing on horizontal gel electrophoresis. A representative *E. coli* DH5α transformant bearing plasmid pGS29 was designated D.1.

Alternatively, plasmid pGS29 was used to transform *Escherichia coli* RV308. A culture of *Escherichia coli* RV308 was deposited in the permanent culture collection of the Northern Regional Research Laboratory (NRRL), United States Department of Agriculture Service, Peoria, IL 61604, on September 28, 1983, and is available under accession number B-15624.

*Escherichia coli* RV308 cells were prepared for transformation as follows. A sample of *E. coli* RV308 cells were grown in L-broth (10 g tryptone, 10 g NaCl, and 5 g yeast extract per liter) to an O.D.₅₉₀ of about 0.5 absorbance units, and the cells were collected by centrifugation. The cell pellet was resuspended in 25 ml of cold 100 mM CaCl₂ and incubated on ice for 25 minutes. The cells were once again collected by centrifugation, and the pellet was resuspended in 2.5 ml of cold 100 mM CaCl₂ and incubated overnight. Competent cells were transformed or stored directly at -70°C until ready for use in transformations.

To transform competent *Escherichia coli* RV308 cells with plasmid pGS29, one hundred microliters of the competent cell suspension in a polypropylene tube were removed from storage at -70°C and allowed to thaw on ice. The cells were gently mixed and five microliters (µl) of a solution of plasmid pGS29 (1 ng/µl) was added and the resulting solution incubated on ice for 30 minutes. The tube was transferred to a 42°C water bath and incubated without shaking for 45 seconds. After the heat-shock treatment, the tube was placed on ice for 2 minutes. The tube was removed from ice and 0.9 ml of S.O.C. medium (2% Bactotryptone; 0.5% yeast extract; 10 mM NaCl; 2.5 mM KCl; 10 mM MgCl₂; 10 mM MgSO₄; 20 mM glucose; in distilled water) at room temperature was added. The solution was incubated for 1 hour at 37°C while shaking at 225 revolutions per minute (rpm). Aliquots of the cell mixture were plated on L-agar (L-broth with 15 grams/liter (g/L) agar) plates containing 50 µg/ml ampicillin, and the plates were incubated at 37°C. Transformants were verified by selection for ampicillin resistance and by plasmid sizing on horizontal gel electrophoresis. Plasmid DNA from the clones was isolated and confirmed by restriction enzyme analysis. Representative transformants of *Escherichia coli* RV308 bearing plasmid pGS29 were designated R.1.

### EXAMPLE 2

### Construction of Plasmid pV-pcrB

### A. Construction of a PCR-Modified/Amplified Fragment

A PCR-modified/amplified fragment that contained the 5' end of the *Escherichia coli gly*A gene altered to have an NdeI site at the ATG start codon and *gly*A encoding DNA inclusive of the BstEII site of the *gly*A gene was constructed.

The DNA oligonucleotide primers used in the amplification were as follows. The forward primer was a 26 base oligonucleotide with the sequence: 5'-GATGCATATGTTAAAGCGTGAAATGA-3' (SEQ. ID. NO: 3).
SEQ. ID. NO: 3 was designed to hybridize across the ATG transcriptional start codon of the *Escherichia coli gly*A gene. The bold type represents the NdeI recognition sequence and the underlined bases were those changed from the original *E. coli gly*A gene sequence.

The reverse oligonucleotide primer was a 15 base pair DNA oligonucleotide with the sequence: 5'-CCGGAGAACCGTGAG-3' (SEQ. ID. NO:4).
SEQ. ID. NO:4 was designed to hybridize immediately downstream of the single BstEII site in the *Escherichia coli gly*A gene sequence. Both oligonucleotides were synthesized on an ABI 380B DNA synthesizer and purified with an Oligonucleotide Purification Cartridge®, (Applied Biosystems Inc., Foster City, CA). Materials necessary for carrying out the PCR reaction with these primers were obtained in a GeneAmp™ DNA Amplification Reagent Kit (Perkin Elmer Cetus, Norwalk, CT).

The reaction conditions were as follows: One nanogram of template DNA (plasmid pGS29); 1.0 mM SEQ. ID. NO:3; 1.0 mM SEQ. ID. NO:4; 200 mM each dATP (deoxyadenosine 5'-triphosphate), dCTP (deoxycytosine 5'-triphosphate), dGTP (deoxyguanosine 5'-triphosphate), and TTP (thymidyl 5'-triphosphate); 1X reaction buffer (10 mM Tris-HCl (pH 8.3); 50 mM KCl; 1.5 mM MgCl₂; 0.01%[w/v] gelatin); and 2.5 units Taq Polymerase in a 100 µl final volume. The reaction mixture (in a 0.5 ml polypropylene tube) was overlaid with 100 µl mineral oil to prevent evaporation was placed in a DNA Thermal Cycler (Perkin Elmer Cetus). Thermocycler settings were as follows: one cycle of 2 minutes at 94°C; thirty cycles of a) 1 minute at 94°C b) 2 minutes at 50°C, and c) 3 minutes at 72°C; one cycle of 7 minutes at 72°C; and a final (storage) cycle at 4°C.

The 403 base pair PCR-modified/amplified DNA fragment was converted to a 380 base pair BstEII-NdeI DNA fragment by digestion with 50 units of BstEII enzyme in a reaction containing 1X BamHI/BstEII buffer (10 mM Tris-HCl (pH 8.0); 5 mM magnesium chloride; 100 mM NaCl; and 1 mM 2-mercaptoethanol) for 2 hours at 60°C. The fragment was gel-isolated as follows. The digested DNA was electrophoresed on an 1.5% agarose gel in 1X TAE buffer (40 mM Tris-Acetate; 1 mM EDTA). The gel was stained in a dilute (1 mg/ml) ethidium bromide solution and the DNA bands visualized under long-wave UV light. The 380 base pair BstEII digested PCR-modified/amplified DNA fragment was located and excised from the gel with a fresh scalpel blade. Elution of the DNA fragment from the gel slice was performed according to the protocol of Maniatis et al.(T. Maniatis, J. Sambrook, and E. F. Fritsch, 1989, Molecular Cloning: A Laboratory Manual Cold Springs Harbor Laboratory Press, pp. 6.28) with minor adjustments. Briefly, the gel slice was put into a dialysis bag with about 200 µl 1/5X TAE buffer, sealed with clips, and electrophoresed in 1/5X TAE buffer for about 3 hours. The contents of the dialysis bag, including a 400 µl wash with 1/5X TAE were mixed with 2.4 ml low salt buffer (0.2 M NaCl; 20 mM Tris-HCl, pH 7.5; 1.0 mM EDTA) and loaded onto a prepared ELUTIP-d column (Schleicher & Schuell, Keene, NH) following the supplied protocol. After washing with low salt buffer the DNA was eluted from the column with two 400 µl volumes of high salt buffer (1.0 M NaCl; 20 mM Tris-HCl, pH 7.5; 1.0 mM EDTA) and precipitated by addition of 800 µl of ice-cold absolute ethanol followed by centrifugation at 14,000 rpm in an Eppendorf 5415C microfuge for 40 minutes. The two air-dried pellets were combined by dissolving in 20 µl TE, pH 7.5 and the solutions were stored at -20°C until ligation. This fragment contained amino-terminal region of the *Escherichia coli gly*A gene modified by PCR to have an NdeI Site at its ATG translation initiation start site.

### B. Construction of a Sau3AI-HphI Linker DNA Fragment

To isolate a Sau3AI-HphI linker fragment two separate digestions and isolations were performed. First, approximately 20 µg of plasmid pUC19 (available from BRL) was digested with 50 units of HphI restriction enzyme (NEB) in a reaction containing 1X HphI buffer (20 mM Tris-acetate (pH 7.9); 50 mM potassium acetate; 10 mM magnesium acetate; and 1 mM dithiothreitol) for 2 hours at 37°C. The appropriate 416 base pair HphI restriction fragment was gel-isolated as in Example 2A. The fragment was then digested with 50 units of Sau3AI restriction enzyme (BM) in a reaction containing 1X Sau3AI buffer (33 mM Tris-acetate (pH 7.9); 10 mM magnesium acetate; 66 mM potassium acetate; 0.5 mM dithiothreitol) for 2 hours at 37°C. The 234 base pair Sau3AI-HphI DNA fragment was gel-isolated as in Example 2A.

### C. Construction of Plasmid pV-pcrA

Approximately 5 µg of plasmid pUC19 DNA were digested with 50 units of restriction enzyme AccI in a reaction containing 1X AccI buffer (33 mM Tris-acetate (pH 7.9); 10 mM magnesium acetate; 66 mM potassium acetate; and 0.5 mM DTT) for 2 hours at 37°C. Linearized plasmid pUC19 was gel isolated by the method described in Example 2A.

Approximately 10 µg of plasmid pGS29 were digested with 50 units of AhaII enzyme (NEB) in a reaction containing 1X AhaII buffer (10 mM Tris-HCl (pH 7.9); 50 mM NaCl; 10 mM MgCl₂; 1 mM DTT; and 100 mg/ml bovine serum albumin) for 2 hours at 37°C. The 2.0 kb AhaII fragment was isolated by the method described in Example 2A.

Three microliters of the 2.0 kb AhaII fragment from plasmid pGS29 and 1 µl of plasmid pUC19 (2.8 kb) linearized with AccI were added to a 15 µl reaction containing 1X ligase buffer and about 5 units of T4 ligase (BM). The reaction mixture was incubated at 14°C for 16 hours. The reaction products were transformed into *Escherichia coli* DH5α cells as described in Example 1. Plasmids from the transformants were screened for orientation of the AhaII fragment in the plasmid. A plasmid containing the BstEII site proximal to the PstI site and distal to the BamHI site of plasmid pUC19 was chosen as plasmid pV-pcrA. Plasmid from this clone was isolated as in Example 1.

To obtain the desired BstEII-BamHI vector backbone fragment, approximately 10 µg of plasmid pV-pcrA were digested with 50 units BamHI enzyme (BM) in a reaction containing 1X BamHI/BstEII buffer for 2 hours at 37°C. BstEII (50 units) was added to the reaction and the temperature raised to 60°C and held for 2 hours. The BstEII-BamHI vector backbone fragment was gel-isolated in accordance with the method of Example 2A.

### D. Final Construction of Intermediate Plasmid pV-pcrB

Intermediate plasmid pV-pcrB was obtained by ligating the BstEII-digested 380 base pair PCR-modified/amplified DNA fragment prepared in Example 2A with the linker fragment prepared in Example 2B and with the vector backbone fragment prepared in Example 2C.

One microliter (about 50 ng) of the vector backbone fragment, 2 µl (about 100 ng) of the Sau3AI-HphI DNA fragment, and 6 µl (about 100 ng) of the BstEII-digested PCR-modified/amplified DNA fragment were added to a reaction containing 1X ligation buffer with about 5 units of T4 ligase. The mixture was incubated at 14°C for 16 hours and the resulting DNA was transformed into *Escherichia coli* DH5α cells by the method described in Example 1. Transformants were screened by restriction enzyme analysis to select a transformant bearing plasmid pV-pcrB. Plasmid pV-pcrB was extracted and purified from that transformant by the method described in Example 1.

### EXAMPLE 3

### Construction of Plasmid pX4

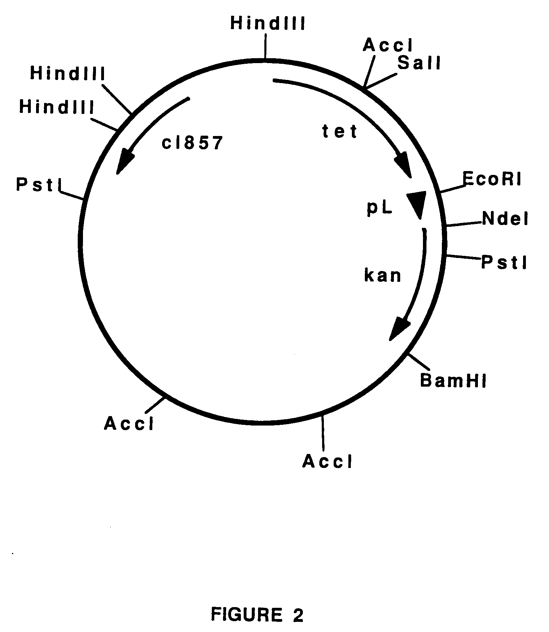

A restriction enzyme site and function map of plasmid pHKY390 is shown in Figure 2. The open reading frame of the kanamycin resistance gene was removed from plasmid pHKY390 as follows. Ten micrograms of plasmid pHKY390 were digested with 50 units of NdeI restriction enzyme in a reaction mixture containing 1X NdeI buffer for 2 hours at 37°C. The digested DNA was precipitated as described above and the resulting DNA pellet was resuspended in 1X BamHI buffer. Fifty units of BamHI restriction enzyme was added, and the mixture was incubated at 37°C for 2 hours. The larger of two NdeI-BamHI DNA fragments of plasmid pHKY390 was gel-isolated according to the procedure described in Example 2A.

The NdeI-BclI DNA fragment, which contained the *Escherichia coli gly*A gene and upstream sequences, was obtained from plasmid pGS29 as follows. Approximately 10 µg of plasmid pGS29 was digested with 50 units NdeI in a reaction containing 1X NdeI buffer for 2 hours at 37°C. The digested DNA was precipitated by the addition of 1/10 volume of 3 M sodium acetate, pH 7.0, and 2.5 volumes of ice-cold absolute ethanol. The DNA was collected by a 30 minute centrifugation. The pellet of DNA was resuspended and dissolved in 1X BclI buffer (10 mM Tris-HCl.(pH 7.5); 10 mM magnesium chloride; 50 mM sodium chloride; and 1 mM dithioerythritol); 50 units of BclI restriction enzyme was added, and the reaction mixture was incubated for 2 hours at 50°C. The 1.73 kb NdeI-BclI DNA fragment was added directly to a ligation mixture containing the gel-isolated NdeI-BamHI DNA fragment from plasmid pHKY390.

The 1.73 kb NdeI-BclI DNA fragment of plasmid pGS29, which contained the *gly*A open reading frame and upstream sequences, was ligated to the large NdeI-BamHI DNA fragment from plasmid pHKY390 as follows. Five microliters (about 50 ng) of the large NdeI-BamHI DNA fragment obtained from plasmid pHKY390 and 5 µl (about 500 ng) of the NdeI-BclI-digested plasmid pGS29 DNA were added to a reaction containing 1X ligase buffer and 5 units of T4 ligase. The resulting mix was incubated at 14°C for 16 hours and the resulting DNA products transformed into *Escherichia coli* DH5α cells as described in Example 1 with the exception that transformation and incubation were carried out at 30°C. Transformants were selected for tetracycline resistance. Plasmids from the resulting tetracycline resistant transformants were screened by restriction enzyme analysis to find transformants that carried a plasmid with the structure of the desired plasmid pX4. Plasmid pX4 DNA was purified from a selected transformant by the method described in Example 1.

### EXAMPLE 4

### Construction of Plasmid pZPl-glyA

To construct plasmid pZPl-glyA the small NdeI-BstEII DNA fragment of plasmid pX4 was replaced with the small NdeI-BstEII DNA fragment from plasmid pV-pcrB. This was accomplished as follows. Plasmid pX4 (10 µg) was digested with NdeI (50 units) in 1X NdeI buffer for 2 hours at 37°C. The DNA was precipitated as in Example 3 and the precipitated DNA was resuspended in 1X BstEII buffer containing 50 units of BstEII. The reaction mixture was incubated at 60°C for 2 hours and the large DNA fragment was gel-isolated according to Example 2A. Approximately 20 µg of pV-pcrB were digested identically to the digestion of plasmid pX4 and the 373 base pair NdeI-BstEII DNA fragment was gel isolated.

The large NdeI-BstEII DNA fragment obtained from plasmid pX4 and the 373 base pair NdeI-BstEII DNA fragment obtained from plasmid pV-pcrB were ligated to form plasmid pZPl-glyA as follows. Two microliters (about 50 ng) of the fragment from vector pX4 and one microliter (about 150 ng) of the 373 base pair fragment from plasmid pV-pcrB were added to a reaction mixture containing 1X ligase buffer and 5 units of T4 ligase. The resulting reaction mixture was incubated at 14°C for 16 hours.

The ligation reaction mixture was prepared for transformation by diluting 5-fold in sterile TE. Using the diluted DNA, competent *Escherichia coli* DH5α cells were transformed essentially as in Example 3. Tetracycline resistant colonies were selected and plasmids from these colonies were sized by horizontal agarose gel electrophoresis. Transformants that contained a plasmid with the size corresponding to that expected for plasmid pZPl-glyA were selected. Plasmids from these transformants were confirmed to have the structure expected for plasmid pZPl-glyA by restriction enzyme analysis. A representative *E. coli* DH5αpZPl-glyA transformant was chosen and designated Dgly.

Plasmid pZPl-glyA was isolated from *Escherichia coli* DH5α /pZPl-glyA and used to transform E. coli RV308 as described in Example 3. Plasmids from these transformants were confirmed to have the structure expected for plasmid pZPl-glyA by restriction enzyme analysis. A representative *E. coli* RV308/pZPl-glyA transformant was chosen and designated Rgly.

To facilitate the activity analysis of the *Escherichia coli* recombinants described above, a high performance liquid chromatography (HPLC) assay was developed using 3'-phenylserine as a substrate for SHMT. The SHMT activity was determined by monitoring the conversion of 3'-phenylserine to benzaldehyde. The reaction mixture in a total volume of 1 ml contained 20 mM of 3'-phenylserine and a suitable amount of the enzyme in 20 mM N, N-bis[2-hydroxyethyl]-2-aminoethanesulfonic acid buffer (BES), pH 7.25. The reaction mixture was incubated at 30°C with gentle shaking and, after 5 minutes, was stopped by addition of 25 µl of 10% H₃PO₄. Any particulates were removed by centrifugation and a small portion (usually 10 µl) of the supernatant fraction was analyzed by HPLC. Benzaldehyde was quantitated by using an Apex ODS 3µ C18 column (Jones Chromatography, Littleton, CO), isocratic elution of 1 ml/min with water/acetonitrile (75/25, v/v) and detection at 279 nm. One unit of enzyme activity is defined as the amount of the SHMT required to cause formation of one µmole of benzaldehyde per minute under the reaction conditions. The specific activity is defined as units per mg of protein. The protein content was determined by the standard method of Bradford using bovine serum albumin as the standard.

### EXAMPLE 5

### Enzyme Assay

The activity of SHMT was determined by monitoring AHHA formation from the condensation reaction of 4-pentenal and glycine using high pressure liquid chromatography (HPLC). The reaction mixture in a total volume of 1 ml contained 45 mM pentenal, 100 mM glycine, an aliquot of enzyme (up to .003 mM), and 0.1 mM pyridoxal-5'-phosphate, in 50 mM phosphate, pH 7.0. The enzymatic reaction was incubated at 15°C with gentle shaking for a reaction time of about 20 to 150 minutes. The product formation was linear to the enzyme concentration and to the reaction time. The enzymatic reaction was stopped by a 20-fold dilution of an aliquot of the mixture (usually 25 µl) with water at 0°C and an immediate treatment with Dabsyl chloride. The Dabsyl chloride derivative of AHHA was quantitated by HPLC using a Beckman ODS-DABS 5µ C18 column (4.6 mm x 25 cm; Beckman Instruments, Fullerton, CA), with a gradient elution of 1.5 ml/minute and with detection at 436 nm. For a typical gradient elution, the mobile phase was constructed from buffer A (4.5% dimethylformamide and 14.5 mM sodium citrate, pH 6.5) and buffer B (4.5% dimethylformamide, 4.5 mM sodium citrate and 70% acetonitrile, pH 6.5) according to the following times: 43-57% B for 7 minutes, 57% B for 1 minute and 57-100% B for 4 minutes. One unit of the enzyme activity is defined as the amount of the SHMT required to cause formation of one µmole of AHHA per min under the reaction conditions. The specific activity is defined as units per µM of SHMT. The protein content was determined by the Bradford method using serum albumin as the standard. Under the conditions as described above, the specific activity of pure SHMT was about 10 units/µM SHMT.

### EXAMPLE 6

### Production of Escherichia coli SHMT

A seed lot was prepared by growing *Escherichia coli* RV308/pZPlglyA (Rgly) at 30°C in L broth with 5 µg/ml tetracycline added, until the desired cell density was reached. One ml aliquots were transferred under sterile conditions to screw-capped freezer vials and preserved in the vapor phase of liquid nitrogen. A vial of the seed lot was removed from storage and the contents transferred equally into 2 flasks each containing 50 ml of L broth with 5 µg/ml tetracycline and incubated at 30°C. The contents of these flasks were used to inoculate a 50 liter stirred reactor containing a modified M-9 glucose-salts medium. This seed vessel was controlled at 30°C, pH 7.0, and dissolved O₂ at greater than 50% until off gas analysis indicated that reasonable cell mass had been achieved. A portion of the broth was then transferred into a 150 liter bioreactor. During the cell mass accumulation phase, temperature was maintained at 30°C and pH controlled at pH 7.0. When fermentation parameters reached a predetermined setpoint (determined by the gas transfer characteristics of each individual bioreactor), the temperature of the fermentor was ramped up to 41°C. At this time recombinant protein synthesis began within the bacterial cells. A slurry of complex amino acids, such as hydrolyzed casein, was bulk fed into the reactor just prior to reaching final temperature. For the remainder of the fermentation, glucose and the complex amino acid slurry were fed at a continuous rate into the reactor. The level fed was sufficient to give and maintain pools of individual amino acids throughout the productive phase. Temperature and pH were controlled throughout the productive phase. The cells containing the correctly folded soluble product were harvested when fermentation parameters such as dissolved oxygen and carbon dioxide evolution rate indicated that fermentation objectives, such as dissolved oxygen rising and carbon dioxide evolution falling, had been met. In 150 liter fermentors, Rgly grew better (i.e. had more total enzyme activity) than Dgly.

### EXAMPLE 7

### Preparation of SHMT

### A. Preparation of Clarified Washed Cell Lysate

Whole cells from an SHMT fermentation were harvested using a Sharples centrifuge and resuspended in an equal volume of phosphate buffer (10 mM sodium phosphate, pH 7.8; 1 mM EDTA; 5 mM PLP). The cell slurry was homogenized using a gaulin press. Cell debris was removed by centrifugation. The centrate was further clarified by microfiltration, using a 0.45 µM filter. A 30 kilodalton molecular weight cut-off ultrafilter was used to concentrate the solution to approximately 30-40 mg/ml of SHMT. The concentrated SHMT solution was then diafiltered against 5 volumes of 100 mM phosphate buffer, pH 7.4, containing 3 mM PLP. The washed cell lysate solution was 0.45 µM filtered and held at -20°C.

### B. Anion Exchange Purification of SHMT

Approximately 17 kilograms of cell paste was suspended in Tris buffer (50 mM Tris; 1 mM EDTA; 0.1 mM PLP; pH 7.8) and passed through a gaulin press to disrupt the cells. The cell debris was removed by centrifugation using a Sharples centrifuge and a resulting centrate was filtered through a Cuno Zeta Plus filter (Cuno). The clarified solution was then loaded onto a 45 x 46 cm column packed with Fast Flow Q anion exchange matrix (Pharmacia Inc.). The column was then washed with six column volumes of Tris buffer. The column was eluted with a linear salt gradient ranging from 0 to 0.5 M KCl over 9 column volumes in the Tris buffer. Fractions were collected and characterized using SDS gel electrophoresis, activity, and total protein using a BCA assay (Bicinchoninic acid, Pearce Chemicals, Inc.). The appropriate fractions were combined, and the mainstream was concentrated using a 30 kilodalton molecular weight cut-off membrane. The concentrated SHMT solution was then diafiltered against 5 volumes of phosphate, PLP containing buffer. The SHMT solution was filtered through a 0.45 µM filter and was held at -20°C.

### EXAMPLE 8

### Preparation of L-erythro-AHHA

One molar glycine, 1 mM pyridoxal 5'-phosphate, and 0.1 mM SHMT (10 g/liter) were mixed together in water at 15°C and pH 7.0. 0.27 equivalents (relative to glycine) of 4-pentenal were added over 5-8 hours. The SHMT was added to the reaction in the form of whole cells (prepared as described in Example 6), a clarified washed cell lysate (prepared as described in Example 7A), or purified by one anion exchange purification step as described in Example 7B.

One to two hours after the 4-pentenal addition was completed, the reaction yield reached a maximum. At this time the pH was adjusted to pH 6.0. If either the clarified washed cell lysate or the one step chromatography purified SHMT was utilized in the reaction, the mixture was concentrated to 1/3-1/4 volumes on a 30 kilodalton molecular weight cut-off ultrafilter membrane, and then washed with 1 volume of 10 mM pH 6.0 phosphate containing 1 mM PLP. The product was in the ultrafilter filtrate. The enzyme, which is retained by the ultrafilter membrane can be recycled for reuse. If whole cells were used in the process, the cells were separated from the reaction mixture by centrifugation at 3500 r.p.m. at 10°C for 15 minutes. The cell pellet was resuspended in the buffer (typically 200-1000 µM PLP, 1 M glycine, 50 mM KPi, pH 7) for reuse.

The product of the reaction and the excess substrate, was removed from either the filtrate or supernatant and analyzed by the DABSYL method for total 2-amino-3-hydroxy acid quantitation (Beckman Dabsylation Kit, Instruction Manual, Beckman Instruments, Altex Division). The yield of L-erythro-AHHA from 4-pentenal was 78-80% and the isomer ratio was 93:7 to 90:10 (erythro:threo). Five to seven percent of the 4-pentenal remained unreacted. The pH was adjusted to pH 6.0 during the concentration steps to minimize threo formation. A pH lower than pH 6.0 precipitated the enzyme.

### EXAMPLE 9

### Effect of pH, temperature and SHMT concentration on yield of L-erythro-AHHA

Condensation reactions were conducted using 200 mM glycine, 40 µM PLP, 50 mM 4-pentenal at various temperatures, pH and SHMT concentrations. The reactions were conducted under batch mode. The results are presented in Table 2. The percent yield represents the maximum observed over the reaction time course, while the percent threo represents the level of threo isomer at the maximum yield point. The data from Table 1 demonstrates that relatively neutral pH and low temperature improves the yield and stereoselectivity, while high SHMT concentrations improve the yield.

**Table 1**

| | [SHMT] (µM) | pH | °C | % yield | % *threo* |
|---|---|---|---|---|---|
| 1. | 100 | 8 | 15 | 75 | 4 |
| 2. | 20 | 8 | 15 | 64 | 4 |
| 3. | 20 | 7 | 15 | 80 | 4.5 |
| 4. | 20 | 7 | 35 | 62 | 12 |

### EXAMPLE 10

### Preparation of L-erythro AHCPA

Glycine (200 mM), pyridoxal 5'-phosphate (1 mM) and SHMT (0.02 mM) were mixed together in water to a volume of 175 ml. The pH was adjusted to 7.0 with 1 N sodium hydroxide. The mixture was cooled to 15°C. 0.27 equivalents of 3-cyanopropanal (relative to glycine) were added to the mixture while the pH was maintained at 7.0 and the temperature maintained at 15°C. The reaction was allowed to proceed for about three hours. The product was removed and analyzed as described in Example 8. The yield of L-erythro AHCPA from 3-cyanopropanal was 58% and the isomer ratio was 98:2 (erythro:threo).

## Claims

1. A process for preparing a 2-amino-3-hydroxy acid of the formula 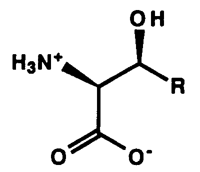 wherein R is C₃-C₆ alkenyl; C₂-C₆ aldehydo; C₃-C₆ alkynyl; C₂-C₆ alkyl substituted by esterified carboxy, C₁-C₆ alkoxy, hydroxy, halo or cyano; phenyl; or furyl, which comprises reacting at a temperature of between about 0°C and about 25°C in an aqueous solution at a pH between about 6.0 and about 8.0 an aldehyde of the formula RCHO (wherein R has the same meaning as defined above) and glycine with *Escherichia coli* serine hydroxymethyltransferase in the presence of pyridoxal 5'-phosphate.

2. A process of Claim 1, wherein the concentration of glycine is from about 0.5 M to about 3 M; the aldehyde is added such that the final ratio of the aldehyde to glycine is about 10:1 to about 2:1; the *Escherichia coli* serine hydroxymethyltransferase is at a concentration from about 10 µM to about 1 mM; the pyridoxal 5'-phosphate is at about a 2-fold to about a 15-fold molar excess of the *Escherichia coli* serine hydroxymethyltransferase; the temperature is from about 10°C to about 20°C; and the pH is from about 6.5 to about 7.5.

3. A process of Claim 2, wherein the concentration of glycine is about 1 M; the aldehyde is added such that the final ratio of the aldehyde to glycine is about 4:1; the *Escherichia coli* serine hydroxymethyltransferase is at a concentration of about 100 µM; the pyridoxal 5'-phosphate is at about 10-fold molar excess of the *Escherichia coli* serine hydroxymethyltransferase; the temperature is about 15°C; and the pH is about 7.0.

4. A process of Claims 1, 2 or 3, wherein the SHMT is obtained from a genetically engineered microorganism containing a plasmid which comprises the *Escherichia coli gly*A gene.

5. A process of Claims 1, 2 or 3, wherein the *Escherichia coli* serine hydroxymethyltransferase is in a clarified washed cell lysate obtained from recombinant *Escherichia coli* cells.

6. A process of Claims 1, 2 or 3, wherein the serine hydroxymethyltransferase has been purified by one step chromatography from a clarified washed cell lysate of recombinant *Escherichia coli* cells.

7. A process of Claims 1, 2 or 3, wherein the *Escherichia coli* serine hydroxymethyltransferase is in intact recombinant *Escherichia coli* cells.

8. A process of Claims 1, 2 or 3 wherein the aldehyde RCHO is selected from the group consisting of C₃-C₆ alkenyl and C₂-C₆ alkyl substituted by esterified carboxy, C₁-C₆ alkoxy, hydroxy, halo, or cyano.

9. A process of Claims 1, 2 or 3, wherein the aldehyde RCHO is selected from the group consisting of succinic semialdehyde methyl ester, succinic semialdehyde ethyl ester, 3-cyanopropanal, and 4-pentenal.

10. A process of Claims 1, 2 or 3, wherein the aldehyde RCHO is 4-pentenal.


SEQUENCE LISTING

(1) GENERAL INFORMATION:
(i) APPLICANT: ELI LILLY AND COMPANY
(B) STREET: Lilly Corporate Center
(C) CITY: Indianapolis
(D) STATE: Indiana
(E) COUNTRY: United States of America
(F) ZIP: 46285
(ii) TITLE OF INVENTION: PROCESS FOR PREPARING 2-AMINO-
3-HYDROXY ACIDS
(iii) NUMBER OF SEQUENCES: 4
(iv) CORRESPONDENCE ADDRESS:
(A) ADDRESSEE: C. M. Hudson
(B) STREET: Erl Wood Manor
(C) CITY: Windlesham
(D) STATE: Surrey
(E) COUNTRY: United Kingdom
(F) ZIP: GU20 6PH
(v) COMPUTER READABLE FORM:
(A) MEDIUM TYPE: Floppy disk
(B) COMPUTER: Macintosh
(C) OPERATING SYSTEM: Macintosh 7.0
(D) SOFTWARE: Microsoft Word 5.1
(2) INFORMATION FOR SEQ ID NO:1:
(i) SEQUENCE CHARACTERISTICS:
(A) LENGTH: 1406 nucleotides
(B) TYPE:nucleic acid
(C) STRANDEDNESS:single
(D) TOPOLOGY:linear
(ii) MOLECULE TYPE:DNA
(xi) SEQUENCE DESCRIPTION:SEQ ID NO:1

T ATG TTA AAG CGT GAA ATG AAC ATT GCC GAT TAT GAT GCC 40

GAA CTG TGG CAG GCT ATG GAG CAG GAA AAA GTA CGT CAG GAA 82

GAG CAC ATC GAA CTG ATC GCC TCC GAA AAC TAC ACC AGC CCC 124

CGC GTA ATG CAG GCG CAG GGT TCT CAG CTG ACC AAC AAA TAT 166

GCT GAA GGT TAT CCG GGC AAA CGC TAC TAC GGC GGT TGC GAG 208

TAT GTT GAT ATC GTT GAA CAA CTG GCG ATC GAT CGT GCG AAA 250

GAA CTG TTC GGC GCT GAC TAC GCT AAC GTC CAG CCG CAC TCC 292

GGC TCC CAG GCT AAC TTT GCG GTC TAC ACC GCG CTG CTG GAA 334

CCA GGT GAT ACC GTT CTG GGT ATG AAC CTG GCG CAT GGC GGT 376

CAC CTG ACT CAC GGT TCT CCG GTT AAC TTC TCC GGT AAA CTG 418

TAC AAC ATC GTT CCT TAC GGT ATC GAT GCT ACC GGT CAT ATC 460

GAC TAC GCC GAT CTG GAA AAA CAA GCC AAA GAA CAC AAG CCG 502

AAA ATG ATT ATC GGT GGT TTC TCT GCA TAT TCC GGC GTG GTG 544

GAC TGG GCG AAA ATG CGT GAA ATC GCT GAC AGC ATC GGT GCT 586

TAC CTG TTC GTT GAT ATG GCG CAC GTT GCG GGC CTG GTT GCT 628

GCT GGC GTC TAC CCG AAC CCG GTT CCT CAT GCT CAC GTT GTT 670

ACT ACC ACC ACT CAC AAA ACC CTG GCG GGT CCG CGC GGC GGC 712

CTG ATC CTG GCG AAA GGT GGT AGC GAA GAG CTG TAC AAA AAA 754

CTG AAC TCT GCC GTT TTC CCT GGT GGT CAG GGC GGT CCG TTG 796

ATG CAC GTA ATC GCC GGT AAA GCG GTT GCT CTG AAA GAA GCG 838

ATG GAG CCT GAG TTC AAA ACT TAC CAG CAG CAG GTC GCT AAA 880

AAC GCT AAA GCG ATG GTA GAA GTG TTC CTC GAG CGC GGC TAC 922

AAA GTG GTT TCC GGC GGC ACT GAT AAC CAC CTG TTC CTG GTT 964

GAT CTG GTT GAT AAA AAC CTG ACC GTT AAA GAA GCA GAC GCC 1006

GCT CTG GGC CGT GCT AAC ATC ACC GTC AAC AAA AAC AGC GTA 1048

CCG AAC GAT CCG AAG AGC CCG TTT GTG ACC TCC GGT ATT CGT 1090

GTA GGT ACT CCG GCG ATT ACC CGT CGC GGC TTT AAA GAA GCC 1132

GAA GCG AAA GAA CTG GCT GGC TGG ATG TGT GAC GTG CTG GAC 1174

AGC ATC AAT GAT GAA GCC GTT ATC GAG CGC ATC AAA GGT AAA 1216

GTT CTC GAC ATC TGC GCA CGT TAC CCG GTT TAC GCA 1252

TAAGCGAAAC GGTGATTTGC TGTCAATGTG CTCGTTGTTC ATGCCGGATG 1302

CGGCGTGAAC GCCTTATCCG GCCTACAAAA CTTTGCAAAT TCAATATATT 1352

GCAATCTCCG TGTAGGCCTG ATAAGCGTAG CGCATCAGGC AATTTTTCGT 1402

TTAT 1406

(2) INFORMATION FOR SEQ ID NO:2:
(i) SEQUENCE CHARACTERISTICS:
(A) LENGTH: 1408 nucleotides
(B) TYPE:nucleic acid
(C) STRANDEDNESS:single
(D) TOPOLOGY:linear
(ii) MOLECULE TYPE:DNA

(ix) FEATURES: reverse complement of SEQ ID NO:1 with NdeI-
BclI compatible ends
(xi) SEQUENCE DESCRIPTION:SEQ ID NO:2

GATCATAAAC GAAAAATTGC CTGATGCGCT ACGCTTATCA GGCCTACACG 50

GAGATTGCAA TATATTGAAT TTGCAAAGTT TTGTAGGCCG GATAAGGCAT 100

TCACGCCGCA TCCGGCATGA ACAACGAGCA CATTGACAGC AAATCACCGT 150

TTCGCTTATG CGTAAACCGG GTAACGTGCG CAGATGTCGA GAACTTTACC 200

TTTGATGCGC TCGATAACGG CTTCATCATT GATGCTGTCC AGCTCGTCAC 250

ACATCCAGCC AGCCAGTTCT TTCGCTTCGG CTTCTTTAAA GCCGCGACGG 300

GTAATCGCCG GAGTACCTAC ACGAATACCG GAGGTCACAA ACGGGCTCTT 350

CGGATCGTTC GGTGCGCTGT TTTTGTTGAC GGTGATGTTA GCACGGCCCA 400

GAGCGGCGTC TGCTTCTTTA ACAATCAGGT TTTTATCAAC CAGATCAACC 450

AGGAACAGGT GGTTATCAGT GCCGCCGGAA ACCACTTTGT AGCCGCGCTC 500

GAGGAACACT TCTACCATCG CTTTAGCGTT TTTAGCGACC TGCTGCTGGT 550

AAGTTTTGAA CTCAGGCTCC ATCGCTTCTT TCAGAGCAAC CGCTTTACCG 600

GCGATTACGT GCATCAACGG ACCGCCCTGA CCACCAGGGA AAACGGCAGA 650

GTTCAGTTTT TTGTACAGCT CTTCGCTACC ACCTTTCGCC AGGATCAGGC 700

CGCCGCGCGG ACCCGCCAGG GTTTTGTGAG TGGTGGTAGT AACAACGTGA 750

GCATGAGGAA CCGGGTTCGG GTAGACGCCA GCAGCAACCA GGCCCGCAAC 800

GTGCGCCATA TCAACGAACA GGTAAGCACC GATGCTGTCA GCGATTTCAC 850

GCATTTTCGC CCAGTCCACC ACGCCGGAAT ATGCAGAGAA ACCACCGATA 900

ATCATTTTCG GCTTGTGTTC TTTGGCTTGT TTTTCCAGAT CGGCGTAGTC 950

GATATGACCG GTAGCATCGA TACCGTAAGG AACGATGTTG TACAGTTTAC 1000

CGGAGAAGTT AACCGGAGAA CCGTGAGTCA GGTGTCCGCC ATGCGCCAGG 1050

TTCATACCCA GAACGGTATC ACCTGGTTCC AGCAGCGCGG TGTAGACCGC 1100

AAAGTTAGCC TGGGAGCCGG AGTGCGGCTG GACGTTAGCG TAGTCAGCGC 1150

CGAACAGTTC TTTCGCACGA TCGATCGCCA GTTGTTCAAC GATATCAACA 1200

TACTCGCAAC CGCCGTAGTA GCGTTTGCCC GGATAACCTT CAGCATATTT 1250

GTTGGTCAGC TGAGAACCCT GCGCCTGCAT TACGCGGGGG CTGGTGTAGT 1300

TTTCGGAGGC GATCAGTTCG ATGTGCTCTT CCTGACGTAC TTTTTCCTGC 1350

TCCATAGCCT GCCAGACTTC GGCATCATAA TCGGCAATGT TCATTTCACG 1400

CTTTAACA 1408

(2) INFORMATION FOR SEQ ID NO:3:
(i) SEQUENCE CHARACTERISTICS:
(A) LENGTH: 26 nucleotides
(B) TYPE:nucleic acid
(C) STRANDEDNESS:single
(D) TOPOLOGY:linear
(ii) MOLECULE TYPE:DNA
(xi) SEQUENCE DESCRIPTION:SEQ ID NO:3

GATGCATATGTTAAAGCGTGAAATGA 26

(2) INFORMATION FOR SEQ ID NO:4:
(i) SEQUENCE CHARACTERISTICS:
(A) LENGTH: 15 nucleotides
(B) TYPE:nucleic acid
(C) STRANDEDNESS:single
(D) TOPOLOGY:linear
(ii) MOLECULE TYPE:DNA
(xi) SEQUENCE DESCRIPTION:SEQ ID NO:4

CCGGAGAACCGTGAG 15